# EUROPÄISCHE PATENTANMELDUNG

(11) **EP 3 157 145 A1**
(43) Veröffentlichungstag der Anmeldung: **19.04.2017**
(21) Anmeldenummer: 15189562.0
(22) Anmeldetag: 13.10.2015
(51) Int. Cl.: H02K 7/09, F04D 17/02, H02K 15/16, A61M 1/10

(54) **ROTOR FÜR EINE PUMPE SOWIE PUMPE UND VERFAHREN ZUR MONTAGE**

(71) Anmelder: Berlin Heart GmbH, 12247 Berlin (DE)
(72) Erfinder: GUNDLACH, Heiko, 10435 Berlin (DE); WINTERWERBER, Kim Peter, Dr., 12357 Berlin (DE)
(74) Vertreter: Pfenning, Meinig & Partner mbB

(57) **Zusammenfassung**

Es werden ein Motor für eine Pumpe und eine Pumpe mit einem Rotor (4) und einem außen liegenden Stator (2) vorgestellt, wobei der Rotor Förderelemente (16) für die Flüssigkeit aufweist und mittels zweier voneinander in Axialrichtung beabstandeter Axiallager (5, 6) gelagert ist. Über eine Steuerspule (27) wird eine Ausgleichsposition des Rotors (4) zwischen den Axiallagern eingestellt. Um die Einstellmöglichkeiten der Rotorposition zu optimieren, ist dafür Sorge getragen, dass die Leitung des magnetischen Flusses wenigstens eines Axiallagers (5) reproduzierbar gelingt.

## Beschreibung

Es wird ein neuartiger Motor für eine Pumpe sowie eine Pumpe mit einem solchen Motor vorgestellt, wobei die Neuerung auf dem Gebiet des Maschinenbaus und der Mechanik bzw. Elektromechanik liegt. Eine Anwendungsmöglichkeit des neuartigen Motors oder der Pumpe liegt beispielsweise auf dem Gebiet der Förderung empfindlicher Flüssigkeiten.

Auf vielen Anwendungsgebieten werden derzeit neuartige Rotorpumpen eingesetzt, die kostengünstig herstellbar sowie gut steuerbar sind und wartungsfrei sowie schonend die kontinuierliche Förderung von Flüssigkeiten erlauben. Dabei soll gleichzeitig eine äußerst reibungsarme und wartungsfreie Lagerung des Rotors erreicht werden. Hierzu werden zunehmend Magnetlager verwendet, die unter anderem im Betrieb auf den Rotor wirkende axiale Schubkräfte wirkungsvoll auffangen können. Allerdings setzt der Einsatz solcher Magnetlager insbesondere dann, wenn der Rotor in zwei solchen Axiallagern gehalten werden soll, eine hohe Genauigkeit bei der Fertigung und Montage der Einzelteile sowie zusätzlich eine Regelung der axialen Lagerkomponente innerhalb eines gegebenenfalls durch Fertigungstoleranzen eingeschränkten Bereiches voraus.

Es wird die Konstruktion eines Motors und/oder einer Pumpe angestrebt, die mit möglichst geringen Fertigungs- und Montagetoleranzen aufbaubar ist.

Es wird zur Lösung der Aufgabe ein Motor für eine Pumpe mit einem radial außen liegenden Stator vorgestellt, der ein Blechpaket aufweist, mit einem radial innen liegenden Rotor, wobei der Rotor axial zu einem Pumpenrohr in zwei voneinander beabstandeten magnetischen Axiallagern gelagert ist. Dieser Motor weist zur Lösung der Aufgabe zudem die Merkmale auf, dass die Position eines Blechpaketes des Stators relativ zu einer Referenzfläche, an der ein Bauteil anliegt, in das der ortsfeste Teil eines ersten Axiallagers integriert ist, maßgeblich, insbesondere ausschließlich, durch ein einziges Positionierelement (ein zwischen einem Blechpaket des Stators und der Referenzfläche angeordnetes Element) festgelegt ist. Der Abstand zwischen dem Blechpaket des Stators und den ortsfesten Lagern bestimmt die axiale Position des Rotors mit.

Es geht bei dem beschriebenen Motor darum, die Position des Stators möglichst einfach und genau gegenüber dem ortsfesten Lagerteil wenigstens eines magnetischen Axiallagers festzulegen. Damit liegt auch der axiale Bewegungsbereich des Rotors, der sich im Stator während des Betriebes ein Stück weit in Axialrichtung bewegen kann, weitgehend fest. Der Stator kann derart positioniert werden, dass seine axiale Mitte zwischen zwei in Axialrichtung beiderseits des Rotors vorgesehenen Axiallagern in einem definierten Abstand angeordnet ist. Somit bleibt für den Rotor, beispielsweise von einer mittigen Ruheposition im Stator aus gesehen, zu beiden Seiten in Axialrichtung gesehen ein bestimmter Spielraum für den Betriebsfall.

Die vorhandene Regelung beeinflusst durch geeignete Ansteuerung einer oder mehrerer Steuerspulen den Magnetkreis zur axialen Lagerung beispielsweise so, dass der Rotor an der Stelle steht, wo sich die axialen Kräfte in beiden Richtungen genau aufheben.

Der Rotor, genauer gesagt insbesondere die im Rotor integrierten und mit ihm fest verbundenen Lagermagnete, wird/werden beidseitig von stationären Lagermagneten axial angezogen, wobei die auf den Rotor wirkende Gesamtkraft vom Magnetabstand in den Axiallagern abhängt, dergestalt, dass der Rotor bei Verlassen dieser Position ohne Gegenmaßnahmen (Regelung mit Steuerspule) unmittelbar zu einem der beiden Lager gezogen würde.

Der Axiallager-Gesamtspalt lässt nur eine bestimmte Maximalverschiebung in Axialrichtung zu, die sich bei einer maximal zulässigen Drehzahl bei gegebenem axial wirkendem Fluidgegendruck einstellt. Um die Pumpe mit einer hinreichend hohen Drehzahl betreiben zu können, sollten die Toleranzen der Gesamtanordnung in Axialrichtung minimiert werden.

Beim Ablösen des Rotors (im Regelfall nur beim Kalibrieren und beim Pumpenstart) von demjenigen Axiallager, das den Rotor vor Inbetriebnahme zu sich hin bis Anschlag angezogen hat, wird die Spule, die auch zur Regelung des Axiallagers vorgesehen ist, weitaus stärker als im normalen Betrieb bis hin zum spezifizierten Grenzbereich bestromt. Optimierte Toleranzen führen auch dazu, dass das Ablösen bei niedrigeren Strömen durch die Steuerspule erfolgt.

Im Betrieb wird der Strom durch die Steuerspule und die Position des Rotors laufend gemessen. Durch eine komplexe Regelung und unter Berücksichtigung der Rotorposition wird der Strom ständig korrigiert.

Die erforderliche Minimierung der mechanischen Toleranzen geschieht dadurch, dass der Stator des Motors möglichst unmittelbar an einer Referenzfläche ausgerichtet wird, an der gleichzeitig auch ein Bauteil ausgerichtet wird, in das der ortsfeste Teil eines ersten Axiallagers integriert ist. Die Ausrichtung des Stators an der Referenzfläche erfolgt durch ein einziges, zwischen einem Blechpaket des Stators und der Referenzfläche angeordnetes Positionierelement. Es ist dort somit nur ein einziges toleranzbehaftetes Teil vorgesehen. Maßgeblich für die Positionierung des Stators ist das Blechpaket, an dem das Positionierelement unmittelbar anliegt, so dass eine Vergussmasse des Stators aus der Toleranzkette herausgenommen ist. Zwischen der Vergussmasse des Stators und anliegenden Teilen können dadurch entsprechende Montagespalte vorgesehen sein.

Das Positionierelement kann aus mehreren verschiedenen Elementen, auch aus unterschiedlichen Materialien, bestehen, die insbesondere miteinander fest verbunden sein können, weist in sich jedoch keine weiteren mechanischen Toleranzen auf.

Es kann beispielsweise vorgesehen sein, dass das Positionierelement den magnetischen Rückfluss vom Statorblechpaket zum ortsfesten Teil des ersten Axiallagers leitet. Das Positionierelement kann beispielsweise ein rohrförmiges, zum Rotor koaxiales Element aus einem Material sein, das den magnetischen Fluss mit geringem Widerstand leitet. Ein solches Rückflusselement wird in vielen Fällen benötigt, um den magnetischen Fluss durch das Axiallager, der axial durch den Rotor, von dort in den Stator und radial außen zurückfließt, in geeigneter Form zu leiten.

Es kann dann auch vorgesehen sein, dass das den magnetischen Rückfluss des ersten magnetischen Axiallagers führende Positionierelement an dem Blechpaket des Stators und an der Referenzfläche spaltfrei anliegt.

Das Positionierelement ist als ein einziges Bauteil vorgesehen, wobei dieses aus einem Stück aus homogenem Material bestehen kann, jedoch auch aus mehreren, fest zusammengefügten Teilen. Vorteilhaft dabei ist lediglich, dass es als Bauteil so fest zusammengefügt ist, dass es nur über einen einzigen Toleranzwert, insbesondere bezüglich der Axialmaße, verfügt. Das Positionierelement kann insgesamt homogen bezüglich der Materialeigenschaften aufgebaut sein, es kann jedoch auch Inhomogenitäten, beispielsweise durch weniger gut magnetisch leitende Schichten, aufweisen.

Ein Motor der oben beschriebenen Art kann konkret beispielsweise so ausgestaltet sein, dass ein erstes Axiallager ein erstes ortsfestes magnetisch aktives Element (ein magnetisch aktives Element kann ein oder mehrere Magnete, ein oder mehrere Rückflussteile oder eine Kombination daraus sein und auch durch nicht magnetische Teile ergänzt werden) aufweist, das magnetisch mit einem ersten magnetisch aktiven Element des Rotors wechselwirkt, wobei der magnetische Kreis von dem ersten magnetisch aktiven Element des Rotors über das erste ortsfeste magnetisch aktive Element, optional ein stutzen- oder bolzenförmiges magnetisch aktives Element mit Flansch (beispielsweise ein Rückflussteil), ein scheibenförmiges, die Referenzfläche bildendes, magnetisch aktives Element (beispielsweise ein Rückflussteil), an dem der Flansch des Stutzens oder des Bolzens plan aufliegt, sowie ein hohlzylindrisches magnetisch aktives Positionierelement (beispielsweise ein Rückflussteil) zum Stator und von dort zum Rotor geschlossen ist und wobei das Blechpaket des Stators in Axialrichtung spaltfrei an dem hohlzylindrischen magnetisch aktiven Positionierelement und dieses in Axialrichtung spaltfrei an der Referenzfläche des scheibenförmigen magnetisch aktiven Elements anliegt. Ein zweites Axiallager kann abweichend aufgebaut sein.

Der gesamte magnetische Kreis wenigstens eines der Axiallager ist damit durch die magnetisch aktiven Teile des Lagers auf der Stator- und Rotorseite, den Rotor selbst, das Blechpaket des Stators und die Rückflusselemente, bestehend aus dem Positionierelement, dem scheibenförmigen Element und dem Stutzen oder Bolzen mit Flansch geschlossen.

Zudem kann noch vorgesehen sein, dass die Steifigkeit mindestens eines Axiallagers zum Abgleich der beiden Axiallager innerhalb eines meist engen Bereiches durch Tausch oder Weglassen eines oder mehrerer Teile möglich ist. So kann der Stutzen oder Bolzen mit Flansch einen Spalt zum benachbarten magnetisch aktiven Element aufweisen. Die Breite des Spaltes (gegebenenfalls durch die Länge des Stutzens oder Bolzens bestimmt) beeinflusst die Steifigkeit des betreffenden Axiallagers.

Der Abgleich der beiden Axiallager ist auch möglich durch geeignete Positionierung des Stutzens oder Bolzens in Axialrichtung, indem er beispielsweise in einem Gewinde in Axialrichtung möglichst fein abgestuft verschiebbar ist. Es kann auch durch die Wahl eines geeigneten Justierstücks aus einer vorgegebenen Auswahl unterschiedlicher Justierstücke mit unterschiedlichen Rückflusseigenschaften eine Justage erfolgen. Es kann aber auch durch einen Schlitz, der durch Verdrehen eines Gegenstückes geschlossen oder überbrückt werden kann, eine Justage erfolgen.

Weiter kann im Aufbau des Motors vorgesehen sein, dass zwischen dem Stator und den Elementen des Pumpengehäuses, insbesondere den Begrenzungselementen einer Strömungskammer, die sich axial an den Stator anschließt, ein oder mehrere Montagespalte vorgesehen sind. Insbesondere die vergossenen Elemente des Stators sollten zu den Begrenzungselementen/Wänden der Strömungskammer einen Abstand in Axialrichtung mit der Funktion eines Montagespaltes zum Toleranzausgleich aufweisen.

Es kann zudem vorgesehen sein, dass das scheibenförmige magnetisch aktive Element einen Durchmesser aufweist, der etwa dem Außendurchmesser des Stators entspricht oder zwischen dem Innendurchmesser und dem Außendurchmesser des Stators liegt. Insbesondere dann, wenn das Positionierelement rohrförmig oder teilweise rohrförmig ausgebildet ist und einen entsprechenden Durchmesser aufweist, können somit die magnetischen Rückflusselemente den magnetischen Fluss des Axiallagers um eine Strömungskammer herum leiten und den magnetischen Kreis schließen. Damit ergibt sich beispielsweise, dass das hohlzylindrische magnetisch aktive Positionierelement eine Strömungskammer mit tangentialem Ausgang umgibt, die sich axial an den Stator anschließt.

Mittels des Bauteils, in das der ortsfeste Teil des ersten Axiallagers integriert ist, ist in einer vorteilhaften Ausgestaltung der ortsfeste Teil des Lagers gegenüber dem Pumpengehäuse fixierbar und insbesondere mit diesem fest verbunden.

Außer dem oben beschriebenen Motor für eine Pumpe soll hier auch eine neue Pumpe beschrieben werden, die sich jeweils durch einen Motor der oben beschriebenen Art auszeichnet, wobei insbesondere der Rotor im ringförmigen Förderraum zwischen dem Rotor und dem Stator Förderelemente zur Förderung eines Fluids trägt.

Die Pumpe ist üblicherweise als Rotorpumpe ausgebildet, wobei der Rotor des Motors unmittelbar Förderelemente zur Förderung eines Fluids tragen kann. Die Flüssigkeit kann durch einen ringförmigen Förderraum zwischen einer Nabe (Rotor oder Leitrad) und Pumpenrohr (Stator) fließen. Axial anschließend an den Rotor kann ein Strömungsleitrad (speichenähnlich im Pumpenrohr gehalten) oder eine Strömungskammer mit tangentialem Auslass vorgesehen sein. Üblicherweise sind der ortsfeste und der rotierende Teil des Axiallagers so angeordnet, dass die magnetisch aktiven Teile nicht mit der geförderten Flüssigkeit in Berührung kommen. Die Lagermagnete sind dazu mit Deckeln abgedeckt, die einen Teil des Gehäuses der Strömungskammer oder eines Strömungsleitrades bilden.

Zusätzlich zu einem Motor und einer Pumpe der oben beschriebenen Art soll auch ein Verfahren zur Montage eines Motors und/oder einer Pumpe vorgestellt werden, das sich dadurch auszeichnet, dass die Rückflussteile und der Stator des Motors im Pumpengehäuse platziert werden und dass danach der Rotor durch eine Regelung der Axiallager in eine stabilisierte Position gebracht wird und dass die Lage der stabilisierten Position bei nicht drehendem Motor zur Kalibrierung insbesondere in Axialrichtung bezüglich der Axiallager ermittelt wird.

Die Position kann mittels Sensoren, insbesondere Wirbelstromsensoren, erfasst werden.

Ist bei dieser Probe-Inbetriebnahme der Pumpe die Lage der Position des Rotors zufriedenstellend, so kann die endgültige Inbetriebnahme ohne weiteres erfolgen. Eine gewünschte Verschiebung der Rotorposition kann auch durch das Einsetzen eines geeigneten Justierstückes in Form eines magnetisch aktiven Stutzens oder Bolzens der oben beschriebenen Art geschehen.

Es kann dazu vorgesehen sein, dass nach der Ermittlung der gewünschten Position bei drehendem oder nichtdrehendem Motor bei der Probe-Inbetriebnahme ein magnetisch aktiver Stutzen oder Bolzen in das scheibenförmige, magnetisch aktive Element eingeführt oder gegen einen dort vorher befindlichen Stutzen oder Bolzen ausgetauscht wird. Auf diese Weise wird ein möglichst großer Einsatzbereich in Hinsicht auf die Fördermenge und Schwankungen der Fördermenge erreicht.

Es ergibt sich damit in Verwirklichung der oben beschriebenen Ideen auch eine Gruppe von Pumpen der oben beschriebenen Art, die sich dadurch auszeichnen, dass wenigstens zwei der Pumpen in Hinsicht auf Abmessungen oder magnetische Rückflusseigenschaften unterschiedliche Stutzen oder Bolzen aufweisen.

Im Folgenden werden Ausführungsbeispiele für den neuen, oben vorgestellten Motor und eine entsprechende Pumpe anhand von Figuren einer Zeichnung gezeigt und anschließend erläutert.

Dabei zeigt
- Fig. 1: in einer perspektivischen Ansicht von außen eine Axialpumpe mit tangentialem Auslass,
- Fig. 2: einen Längsschnitt durch eine Axialpumpe mit tangentialem Ausgang ähnlich der in Figur 1 dargestellten, jedoch ohne Gehäuse, sowie

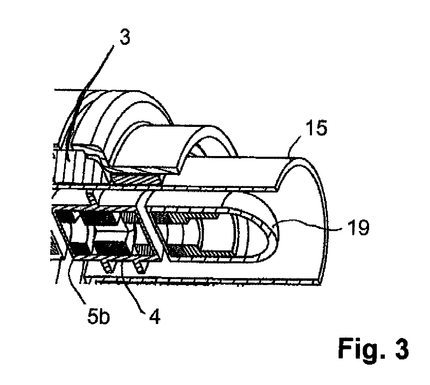

- Fig. 3: eine perspektivische Ansicht eines längsgeschnittenen Teils der Pumpe aus Figur 2 auch ohne Gehäuse.

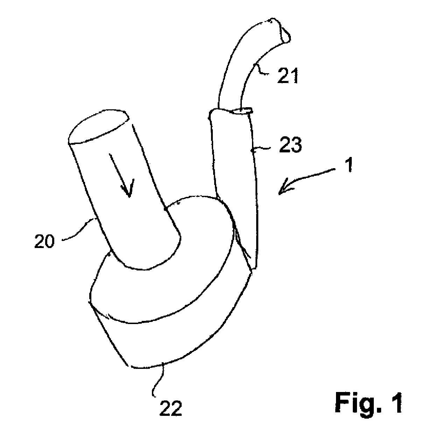

Figur 1 zeigt eine typische oben beschriebene Pumpe, wobei durch den Pumpeneinlass 20 die Flüssigkeit angesaugt wird. Hier schließt sich die Pumpenbasis 22 an, die im inneren eine Strömungskammer aufweist. Die Flüssigkeit wird von der Strömungskammer über den tangential außen liegenden Pumpenauslass 23 in Richtung zu einem Ablauf 21 wieder ausgestoßen.

Figur 1 ist beispielsweise eine Pumpe, wie sie typischerweise als Blutpumpe bei Patienten intrakorporal (im Körper des Patienten implantiert, bei der Implantation wird zuvor mit geeignetem Werkzeug ein rundes Loch in die Herzwand geschnitten) eingesetzt wird, wobei durch eine erste Kanüle über den Pumpeneinlass 20 Blut beispielsweise aus einer Herzkammer angesaugt und über eine zweite Kanüle 21 in Richtung zu einem Blutgefäß wieder ausgestoßen wird.

Die Pumpe 1 weist üblicherweise einen Elektromotor als Antrieb auf, der in die Pumpe integriert ist und mittels einer nicht im Einzelnen dargestellten Steuereinrichtung angesteuert wird. Bei dem Elektromotor kann es sich um einen bürstenlosen Elektromotor handeln, der mit pulsweitenmodulierten Signalen angesteuert wird.

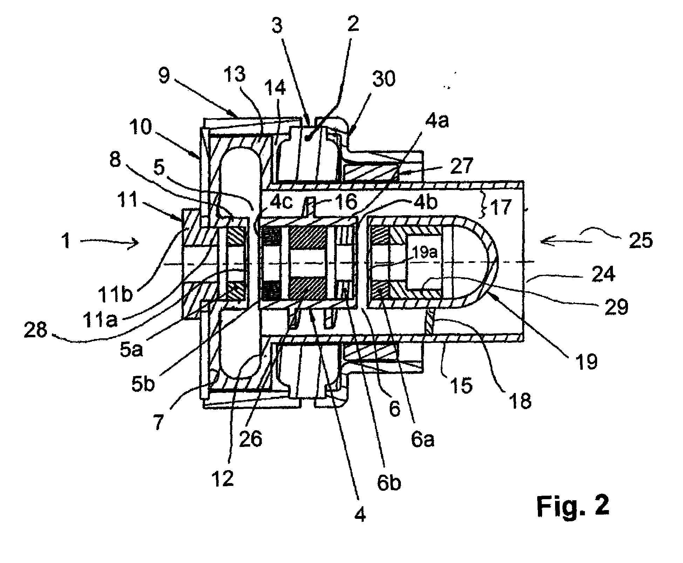

Figur 2 zeigt einen Längsschnitt der Pumpe ohne Gehäuse, wobei auf der rechten Seite die Pumpeneinlassöffnung 24 angeordnet ist, an der in Richtung des Pfeils 25 Flüssigkeit einströmt. Die Flüssigkeit fließt durch das Pumpenrohr 15 durch den ringförmigen Förderraum 17 zwischen der Innenwand des Pumpenrohrs 15 und der Nabe eines sogenannten Vorleitrades 19 (dieses ist ein Strömungsleitrad, dessen Nabe speichenähnlich radial in der Mitte gehalten wird, wobei die Speichen als Flügel ausgebildet sind und Strömungsleiteigenschaften aufweisen) bzw. zwischen der Innenwand des Pumpenrohres 15 und der Nabe des Rotors 4, der sich axial an das Vorleitrad 19 anschließt. Der Rotor 4 weist Förderelemente 16, beispielsweise in Form von schraubenartig verlaufenden Schaufeln, auf, die die Flüssigkeit in Axialrichtung 25 fördern. Zudem wird die Flüssigkeit in axialer Richtung beschleunigt und zur Strömungskammer 13 befördert. Sekundär wird die Flüssigkeit auch tangential beschleunigt, so dass sie mit Hilfe der Strömungskammer 13 in den in Fig. 1 dargestellten tangentialen Pumpenauslass 23 geleitet wird.

Der Antriebsmotor, der einen radial außen liegenden Stator 2 sowie einen radial innen liegenden Rotor 4 aufweist, ist in den Pumpenaufbau integriert. Der Magnetspalt des Elektromotors liegt dabei in dem ringförmigen Förderraum 17, durch den auch die Flüssigkeit strömt.

Mit 26 sind die Rotormagnete bezeichnet, die mit dem Statorfeld zum Antrieb der Pumpe wechselwirken.

Der Rotor weist ein zylindrisches Gehäuse auf, das einen Rohrteil 4a sowie zwei Abschlussdeckel 4b, 4c aufweist. Diese dichten das Innere des Rotors gegen die zu fördernde Flüssigkeit ab. Auch das Vorleitrad 19 weist ein Gehäuse auf, das durch einen Deckel 19a abgeschlossen ist.

Die Pumpenanordnung weist zwei Axiallager 5, 6 auf, die an den beiden einander gegenüberliegenden axialen Enden des Rotors 4 angeordnet sind. Jedes der Axiallager 5, 6 weist ein ortsfestes magnetisch aktives Teil 5a, 6a, insbesondere einen ortsfesten Magneten, sowie ein im Rotor befindliches magnetisch aktives Teil 5b, 6b auf, das ebenfalls als Magnet, jedoch auch als den Magnetfluss gut leitendes Bauteil ausgebildet sein kann. Die ortsfesten Lagermagneten 5a, 6a ziehen beide jeweils den ihnen zugewandten Rotormagneten an, wobei die Kraft vom Abstand abhängig ist. Dies führt dazu, dass es keine stabile Axialposition gibt und das Magnetlager ständig nachgeregelt werden sollte. Minimale Verschiebungen weg von der anzustrebenden Position hätten ohne Nachregelung zur Folge, dass sich der Rotor weiter von dieser Position weg bis zu einem der Axiallager hin bewegen würde.

Es ist eine Steuerung mit einer Steuerspule 27 vorgesehen, die im magnetischen Flusskreis des Magnetlagerflusses des Axiallagers 6 liegt bzw. Einfluss auf den magnetischen Fluss ausübt und im vorliegenden Fall den Magnetfluss des Axiallagers 6 stärken oder schwächen kann. Die Position des Rotors wird durch die Regelung derart eingestellt, dass die Steuerspule 27 den Rotor axial in einer dafür vorgesehenen Position zwischen den ortsfesten Lagerelementen 5a, 6a hält. Um zuverlässig eine günstige ausgeglichene Position des Rotors 4 beim Bau und bei der Montage der Pumpe zu erreichen, ist es wichtig, dass die Bauteile der Pumpe, die einen Einfluss auf die magnetischen Flüsse haben, in vorhersehbarer und reproduzierbarer Weise zusammengesetzt werden können. Insbesondere müssen auf der Seite des Lagers 5 die magnetischen Flusseigenschaften der Lagermagnete 5a, 5b, des Blechpaketes 3, des Positionierelementes 9 und des scheibenförmige Elementes 10 hinreichend genau reproduzierbar sein.

Insbesondere muss auch der Abstand des Blechpakets 3 von dem ortsfesten Lagermagnet 5a in Axialrichtung 25 möglichst genau festgelegt sein. Aus diesem Grund ist die Konstruktion der Pumpe so gestaltet, dass das Blechpaket 3 unmittelbar auf dem Positionierelement 9 in Axialrichtung spaltfrei aufliegt und dieses auf dem scheibenförmigen Element 10 ebenfalls spaltfrei aufliegt, und zwar auf der Referenzfläche 7, die dem Pumpeninneren zugewandt ist und auf der auch die Strömungskammer 13 mit dem fest zur Referenzfläche beabstandeten Lagermagnet 5a unmittelbar aufliegt.

In der Strömungskammer 13, genauer in einen nach innen ragenden Stutzen 8 des Gehäuses, ist der ortsfeste Lagermagnet 5a integriert, d. h., er ist in diesem festgelegt, beispielsweise eingegossen. Über dem Lagermagneten 5a ist ein Deckel 28 angeordnet, der den Lagermagneten vom Inneren der Strömungskammer 13 trennt. Der Deckel kann hierbei auch in das Gehäuse fest integriert sein. Die beiden Lagermagnete 5a, 5b sind somit durch die beiden Deckel 4c, 28 und den sich ergebenden axialen Lagerspalt voneinander getrennt.

Damit das Blechpaket 3 auch unmittelbar und spaltfrei an das Positionierelement 9 zu liegen kommt, ist zwischen dem Verguss des Stators 2 und dem Gehäuse der Strömungskammer 13 ein Montagespalt 14 vorgesehen.

Bei der Inbetriebnahme der Pumpe nach dem Zusammenbau kann zunächst die Regelung der Steuerspule 27 bei nicht drehendem Rotor in Betrieb genommen werden.

Zur Korrektur dieser Ausgangsposition kann ein Justierstück in Form eines Stutzens 11 in einer zentrischen Öffnung des scheibenförmigen Elements 10 in unmittelbarer Nähe des ortsfesten Lagermagneten 5a vorgesehen werden. Der Stutzen 11 weist dann durch seine Länge bzw. die Länge seines Rohrelements 11a einen definierten Abstand (z. B. Luftabstand) zu dem Lagermagneten 5a auf, wobei das Flanschelement 11b unmittelbar auf dem scheibenförmigen Element 10 aufliegen kann. Der Stutzen 11 besteht aus einem Material, das den magnetischen Fluss gut leitet, so dass der magnetische Fluss an dieser Stelle durch Veränderung des Abstandes zwischen Stutzen 11 und Lagermagnet 5a beeinflusst werden kann. Es kann aber auch eine Justiervorrichtung in Form eines Gewindes zwischen dem Stutzen 11 und dem scheibenförmigen Element 10 vorgesehen sein, über das der Stutzen 11 in Axialrichtung justiert werden kann. Die eingestellte Position des Rotors 4 kann beispielsweise über Wirbelstromsensoren oder andere Abstandssensoren festgestellt werden.

Die optimierte Gestaltung der Rückflussteile des Magnetlagers hat auch einen Einfluss auf die Kraft, die notwendig ist, um den Rotor durch einen Impuls der Steuerspule 27 anfänglich von einem der Axiallagermagnete, an dem der Rotor angezogen worden ist, loszureißen. Durch eine geeignete Einstellung des Flusses kann auch der hierfür notwendige Spannungsimpuls in der Steuerspule begrenzt werden.

Am einlassseitigen Ende des Rotors 4 weist dieser den rotierenden Lagermagneten 6b auf, der mit dem ortsfesten Lagermagneten 6a im Vorleitrad 19 wechselwirkt. Im Vorleitrad 19 ist zudem axial hinter dem Lagermagneten 6a ein Flussleitelement 29 vorgesehen, in das der Fluss aus dem radial außerhalb des Pumpenrohrs 15 liegenden Rückflusselement 30 eintritt. Dieses Element 30 leitet den Fluss vom Blechpaket 3 axial in Richtung Einlass, von da radial nach innen über die Steuerspule bis zu dem Flussleitelement 29, durch den ortsfesten Lagermagneten 6a, den rotierenden Lagermagneten 6b, die Antriebsmagneten 26 und zurück in das Blechpaket 3.

Das Vorleitrad 19 ist mittels speichenähnlicher Stege 18 in dem Pumpenrohr gehalten, so dass der zu fördernden Flüssigkeit im ringförmigen Förderraum 17 ein definierter Widerstand, der im Idealfall zu günstigen Strömungsverhältnissen führt, entgegengesetzt wird.

## Patentansprüche

1. Motor (2, 3, 4) für eine Pumpe (1) mit einem radial außen liegenden Stator (2), der ein Blechpaket (3) aufweist, und mit einem radial innen liegenden Rotor (4), wobei der Rotor in zwei voneinander in Axialrichtung des Rotors beabstandeten magnetischen Axiallagern (5, 6) gelagert ist, **dadurch gekennzeichnet, dass** die Position des Stators (2) relativ zu einer Referenzfläche (7), an der ein Bauteil (8) anliegt, in das der ortsfeste Teil (5a) eines ersten Axiallagers (5) integriert ist, ausschließlich durch ein einziges, zwischen einem Blechpaket (3) des Stators (2) und der Referenzfläche (7) angeordnetes Positionierelement (9) festgelegt ist.

2. Motor nach Anspruch 1, **dadurch gekennzeichnet, dass** das Positionierelement (9) den magnetischen Rückfluss vom Statorblechpaket (3) zum ortsfesten Teil (5a) des ersten Axiallagers (5) leitet.

3. Motor nach Anspruch 1 oder 2, **dadurch gekennzeichnet, dass** das den magnetischen Rückfluss des ersten magnetischen Axiallagers (5) führende Positionierelement (9) an dem Blechpaket (3) des Stators (2) und an der Referenzfläche (7) spaltfrei anliegt.

4. Motor nach Anspruch 1, 2 oder 3, **dadurch gekennzeichnet, dass** ein erstes Axiallager (5) ein erstes ortsfestes magnetisch aktives Element (5a) aufweist, das magnetisch mit einem ersten magnetisch aktiven Element (5b) des Rotors (4) wechselwirkt, wobei der magnetische Kreis von dem ersten magnetisch aktiven Element (5b) des Rotors (4) über das erste ortsfeste magnetisch aktive Element (5a), ein scheibenförmiges, die Referenzfläche (7) bildendes, magnetisch aktives Element (10) sowie ein hohlzylindrisches magnetisch aktives Positionierelement (9) zum Stator (2) und von dort zum Rotor (4) geschlossen ist und wobei das Blechpaket (3) des Stators in Axialrichtung (25) spaltfrei an dem hohlzylindrischen magnetisch aktiven Positionierelement (9) und dieses in Axialrichtung spaltfrei an der Referenzfläche (7) des scheibenförmigen magnetisch aktiven Elements (10) anliegt.

5. Motor nach Anspruch 1 oder einem der folgenden, **dadurch gekennzeichnet, dass** an einem scheibenförmigen magnetisch aktiven Element (10), das die Referenzfläche (7) aufweist, ein zentraler, sich in Richtung auf das erste ortsfeste magnetisch aktive Element (5a) hin erstreckender magnetisch aktiver Stutzen (11) angeordnet ist.

6. Motor nach Anspruch 1 oder einem der folgenden, **dadurch gekennzeichnet, dass** zwischen dem scheibenförmigen magnetisch aktiven Element (10), das die Referenzfläche (7) bildet, und dem ersten ortsfesten magnetisch aktiven Element (5a) ein magnetisch aktiver Stutzen (11) als gesondertes Bauteil mit einem Rohrelement (11a) und einem an dem scheibenförmigen magnetisch aktiven Element (10) spielfrei anliegenden Flanschelement (11b) vorgesehen ist.

7. Motor nach Anspruch 1 oder einem der folgenden, **dadurch gekennzeichnet, dass** zwischen dem Stator (2) und den Elementen des Pumpengehäuses, insbesondere den Begrenzungselementen (12) einer Strömungskammer (13), die sich axial an den Stator (2) anschließt, ein oder mehrere Montagespalte (14) vorgesehen sind.

8. Motor nach Anspruch 1 oder einem der folgenden, **dadurch gekennzeichnet, dass** das scheibenförmige magnetisch aktive Element (10) einen Durchmesser aufweist, der etwa dem Außendurchmesser des Stators entspricht oder zwischen dem Innendurchmesser und dem Außendurchmesser des Stators (2) liegt.

9. Motor nach Anspruch 1 oder einem der folgenden, **dadurch gekennzeichnet, dass** das hohlzylindrische magnetisch aktive Positionierelement (9) eine Strömungskammer (13) umgibt, die sich axial an den Stator (2) anschließt.

10. Motor nach Anspruch 1 oder einem der folgenden, **dadurch gekennzeichnet, dass** ein ortsfester Magnet (5a) des ersten Axiallagers (5) relativ zum Pumpengehäuse (12, 13, 15) fixiert, insbesondere an diesem befestigt ist.

11. Pumpe mit einem Motor nach einem der Ansprüche 1 bis 10, wobei insbesondere der Rotor im Radialspalt zwischen dem Rotor (4) und dem Stator (2) Förderelemente (16) zur Förderung eines Fluids trägt.

12. Verfahren zur Montage eines Motors (2, 3, 4) und/oder einer Pumpe, beispielsweise einer Herzpumpe (1), nach einem der Ansprüche 1 bis 11, **dadurch gekennzeichnet, dass** die Rückflussteile (9, 10) und der Stator (2) des Motors im Pumpengehäuse (12, 13, 15) platziert werden und dass danach der Rotor (4) durch eine Regelung wenigstens eines Axiallagers (5, 6) in eine stabilisierte Position gebracht wird und dass die Lage der stabilisierten Position bei nichtdrehendem Motor zur Kalibrierung insbesondere in Axialrichtung bezüglich der Axiallager (5, 6) ermittelt wird.

13. Verfahren nach Anspruch 12, **dadurch gekennzeichnet, dass** nach der Ermittlung der Lage der stabilisierten Position bei drehendem oder nichtdrehendem Motor ein magnetisch aktiver Stutzen (11) in das scheibenförmige, magnetisch aktive Element (10) eingeführt oder gegen einen dort vorher befindlichen Stutzen (11) ausgetauscht wird, um eine Ausgangslage des Rotors zu verändern.

14. Gruppe von Pumpen, beispielsweise Herzpumpen (1), montiert in einem Verfahren gemäß Patentanspruch 12 oder 13, **dadurch gekennzeichnet, dass** wenigstens zwei der Pumpen unterschiedliche magnetisch aktive Stutzen (11) aufweisen.

15. Pumpe zur Herzunterstützung, insbesondere zur intrakorporalen Anwendung, enthaltend einen Motor nach einem der Ansprüche 1 bis 10 und/oder nach Anspruch 11.
